# EUROPEAN PATENT SPECIFICATION

(11) **EP 0 996 380 B1**
(45) Date of publication and mention of the grant of the patent: **05.01.2005**
(21) Application number: 98935803.1
(22) Date of filing: 20.07.1998
(51) Int. Cl.: A61B 18/00

(54) **ELECTROSURGERY DEVICE**
ELEKTROCHIRURGISCHE VORRICHTUNG
DISPOSITIF D'ELECTROCHIRURGIE

(30) Priority: 18.07.1997 US 896398
(43) Date of publication of application: 03.05.2000
(73) Proprietor: Medtronic, Inc., Minneapolis, Minnesota 55432-5604 (US)
(72) Inventor: MULIER, Peter, M., J., Stillwater, MN 55082 (US); HOEY, Michael, F., Shoreview, MN 55126 (US)
(74) Representative: Hughes, Andrea Michelle
(86) International application number: PCT/US1998/014930
(87) International publication number: WO 1999/003414

(56) References cited:
- WO-A-95/31144
- WO-A-97/05829
- US-A- 5 300 087
- US-A- 5 364 394
- US-A- 5 417 709

## Description

This invention relates to medical instruments, and more particularly to electrosurgical devices such as devices for manipulating tissue as, for example, by cutting the tissue.

High-frequency alternating current was used to cut and coagulate human tissue as early as 1911. Current generators and electrode tipped instruments then progressed such that electrosurgical instruments and current generators are available in a multitude of configurations for both open procedures and endoscopic procedures, with microprocessor-controlled currents typically on the order of 500KHz. Radiofrequency (RF) catheter ablation of brain lesions began in the 1960s, and RF ablation of heart tissue to control supraventricular tachyarrhythmias began in the 1980s. Thus, electrical energy, including but not limited to RF energy, is a known tool for a variety of effects on human tissue, including cutting, coagulating, and ablative necrosis, with and as a part of electrically conductive forceps. Bipolar and monopolar currents are both used with electrosurgical forceps. With monopolar current, a grounding pad is placed under the patient. A recent example of an electrically energized electrosurgical device is disclosed in U.S. Patent No. 5,403,312 issued on April 4, 1995 to Yates et al.

US-A-5300087 discloses an electrosurgical tissue sealing device having infusion openings in one of its jaws.

An object of the present invention is to provide an electrosurgery medical device which may and also may not be a forceps.

Document US-A-5 417 709 discloses an electrosurgical device comprising, in essence, two jaws with solution infusion openings defined along portions of both jaws, the openings receiving an electrically conductive solution from a solution supply means, and an electrical conductor which is connected to the end tips of the jaws. The end tips are not electrically connected to the solution supply means from which the apertures receive the conductive solution.

Another object of the present invention is to provide an electrosurgery device such as a forceps that seals tissue by a unique flow of an electrolytic fluid or solution to the manipulating portions of the device in combination with energization of the solution with electrical energy.

The invention provides an electrosurgical medical device, comprising in combination:
co-operating jaws each including a jaw portion for manipulating tissue;
solution supply means for supplying electrolytic solution to the jaw portions;
solution infusion openings defined along the jaw portions of the jaws, the solution infusion openings being constructed and arranged for receiving an electrically conductive solution from the solution supplymeans and infusing the solution through the openings and along the jaw portions into tissue manipulated by the jaw portions; and
an electrical conductor electrically connected to the solution supply means for conducting electrical energy to the electrically conductive solution in contact with the manipulated tissue.

The effect of the solution and energy may be enhanced with pressure. The solution is brought into contact with and infuses the tissue. The solution may include saline as well as other non-toxic and toxic electrolytic solutions, and may be energized with RF electrical energy. The body of the device itself may or may not be energized.

The solution provides at least in part the beneficial functions and effects of the instrument. As preferred, pressure on the tissue is applied, and most preferably the effect of pressure is optimized, as by applying pressure across the tissue to be effected that is substantially uniform.

Another object of the invention is to provide an electrosurgery medical device as described in which tissues are sealed against flow of fluids including air. With the invention, for example, lung tissue is aerostatically and hemostatically sealed, with the tissue adjacent the sealed tissue retaining blood and air.

Another object of the invention is to provide an electrosurgery medical device that may take the form of open surgery forceps of a variety of specific forms, or endoscopic forceps, also of a variety of forms.

A further object of the invention is to provide an electrosurgery medical device as described, in which the electrolytic solution by which the instrument functions is infused from the device onto and/or into the tissue along the operative portions of the device. With and without applied pressure, the solution coagulates and additionally seals tissue, as a result of being energized by RF energy, and also envelopes the operative portions of the device in solution all during manipulation of tissue, substantially completely preventing adherence between the instrument and tissue, substantially without flushing action.

In a principal aspect, then, the invention takes the form of an enhanced solution-assisted electrosurgery medical device comprising, in combination, co-operating device jaws including jaw portions for manipulating tissue, and a plurality of solution infusion openings defined and spaced along each of the jaw portions, for receiving solution and infusing solution onto and into the tissue along said jaw portions. While the device is contemplated with and without grooves, as preferred, the device further comprises at least one, and most preferably, many, longitudinal grooves along at least one and most preferably, both, of the jaw portions. Also most preferably, the solution infusion openings are located on the inside faces of the jaw portions, adjacent to and most preferably in the groove or grooves. The solution exiting the openings separates substantially all the operative surfaces of the device from tissue, substantially completely preventing adherence between the operative surfaces and tissue. The solution also aids in coagulation.

Coagulation aside, the invention causes hemostasis, aerostasis, and more generally, "omnistasis" of substantially any and all liquids and gases found in tissue being treated, such as lymphatic fluids and methane, as well as blood and air. These broader effects are understood to result from such actions as shrinkage of vascalature with and without coagulation, and without desiccation and carbonization.

Also as preferred, the operative portions of the device may take the form of a circular, semicircular or other regular and irregular geometric shape, to contain and/or isolate tissue to be affected and perhaps resected. As an example, with an enclosed geometric shape such as a circle, tissue surrounding lesions and/or tumors of the lung may be aerostatically and hemostatically sealed, resulting in an isolation of the lesions and/or tumors for resection. Lung function is retained. For adaption to unique tissue geometries, the operative portions of the device may be malleable, to be manipulated to substantially any needed contour. For procedures including resection, the device may include an advanceable and retractable blade, or additional functional structures and features.

In accordance with a preferred embodiment, a plurality of longitudinal grooves is provided along said jaw portions; there is also provided
a pivot for pivoting movement of said jaws into contact with each other and away from contact with each other;
the plurality of solution infusion openings is defined and spaced along each of the jaw portions for manipulating tissue, said solution infusion openings located in said plurality of longitudinal grooves, for receiving solution and infusing solution into the tissue along said jaw portions;
a solution supply to said plurality of solution infusion openings is provided for supplying solution at a rate in a range of .01 to 100 cc/min;
an electrical supply of electrical energy is provided sufficient to affect tissue, to said solution supply;
and there is also provided
an extended shaft connected to said jaws for endoscopic use of said device.

These and other objects, advantages and features of the invention will become more apparent upon a reading of the following detailed description of preferred embodiments of the invention, which is given by way of example only, and reference to the drawing which accompanies this description.

The accompanying drawing includes a variety of figures. Like numbers refer to like parts throughout the drawing. In the drawings:
Fig. 1 is a schematic diagram of the key elements of an electrical circuit according to the invention;
Fig. 2 is a perspective view of an endoscopic forceps according to an embodiment of the invention;
Fig. 3 is a detail view of a portion of the forceps of Fig. 2; and
Fig. 4 is a perspective view of a modification of the embodiment of Fig. 2;
Fig. 5 is a second modification of the embodiment of Fig. 4, shown partially broken away;
Fig. 6 is a perspective view of an open surgery forceps according to an embodiment of the invention;
Fig. 7 is a detail view of a portion of the forceps of Fig. 6, partially broken away;
Fig. 8 is a schematic view of preferred saline supply equipment for the invention;
Fig. 9 is a perspective view of a portion of the jaws of an alternative device;
Fig. 10 is a perspective view similar to Fig. 9 of another alternative device;
Fig. 11 is a cross-sectional view along line 11-11 of Fig. 9; and
Fig. 12 is a perspective view of yet another alternative device.

Electrosurgery uses electrical energy to heat tissue and cause a variety of effects such as cutting, coagulation and ablative necrosis. The heat arises as the energy dissipates in the resistance of the tissue. The effect is dependent on both temperature and time. Lower temperatures for longer times often yield the same effect as higher temperatures for shorter times. Normal body temperature is approximately 37°C. No significant long-term effect is caused by temperatures in the range of 37°C to 90°C. In the range of 41°C to 44°C, cell damage is reversible for exposure times less than several hours. In the range of 45°C to 49°C, cell damage becomes irreversible at increasingly short intervals. The following table states expected effects at higher temperatures:

| Temperature (°C) | Effect |
|---|---|
| 50-69 | Irreversible cell damage - ablation necrosis. |
| 70 | Threshold temperature for shrinkage of tissue. (Some collagen hydrogen bonds break at 60-68; those with cross-linkages break at 75-80.) |

| | |
|---|---|
| 70-99 | Range of coagulation. Hemostasis due to shrinkage of blood vessels. |
| | |
| 100 | Water boils. |
| | |
| 100-200 | Desiccation as fluid is vaporized. Dependent on the length of time during which heat is applied, carbonization may occur, and at higher temperatures, occurs quickly. |

This table is not intended as a statement of scientifically precise ranges above and below which no similar effects will be found, and instead, is intended as a statement of generally accepted values which provide approximations of the ranges of the stated effects.

As a consequence of the foregoing effects, preferred "soft" coagulation occurs at temperatures slightly above 70°C. Heat denatures and shrinks tissues and blood vessels, thereby leading, as desired, to control of bleeding. Cells are generally not ruptured. "Soft" coagulation is generally assured with voltages below 200 peak Volts. Sparks are avoided. "Forced" coagulation can be accomplished with bursts of electrical energy. Electric arcs are generated. Deeper coagulation is achieved, at the cost of some carbonization and an occasional cutting effect. Spray coagulation is also possible. Tissue cutting occurs by desiccation, when the concentration of electrical energy, also referred to here as energy density, is acute, and the temperature of tissue is raised above 100°C.

For both coagulation and cutting by electrical energy, a sine wave waveform is employed, with a frequency of about 500 kHz. For cutting, increasing voltage to as much as 600 peak Volts leads to higher spark intensity which results in deeper cuts. Frequencies above 300,000 Hz avoid stimulating nerve and muscle cells, and generally assure that the effect on tissue is substantially purely thermal.

In contrast with the RF energy tissue-cutting electrosurgery tools of the past, significant purposes of the present invention are to provide a mechanism of avoiding desiccation of tissue at the electrode/tissue interface and to achieve sealing of tissues. By "sealing", the effects of hemostasis, or arresting of bleeding; "aerostasis", or arresting of the passage of air; and closure of tissues such as blood vessels against larger-scale passage of blood, among other effects, are intended. More specifically, the effect of sealing at the cellular level is a primary focus, as is sealing at the vascular level.

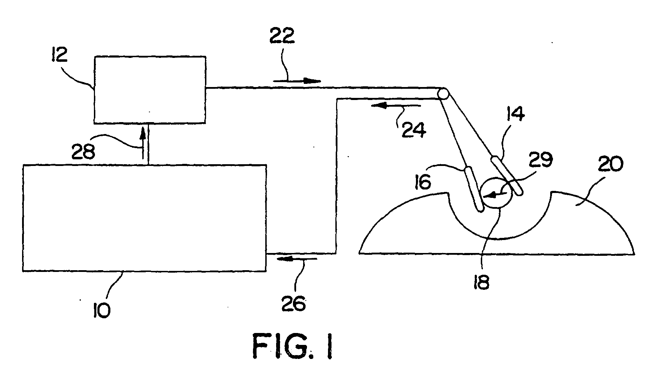

Referring to Figure 1, key elements of a preferred electrical circuit according to the invention include an electrosurgical unit 10, a switch 12, and electrodes 14, 16. An effect is created on tissue 18 of a body 20. One electrode such as electrode 14 acts as a positive or active electrode, while the other such as electrode 16 acts as a negative or return electrode. Current flows directly from one electrode to the other primarily through only the tissue, as shown by arrows 22, 24, 26, 28, 29. No pad is needed under the patient. This is a bipolar configuration.

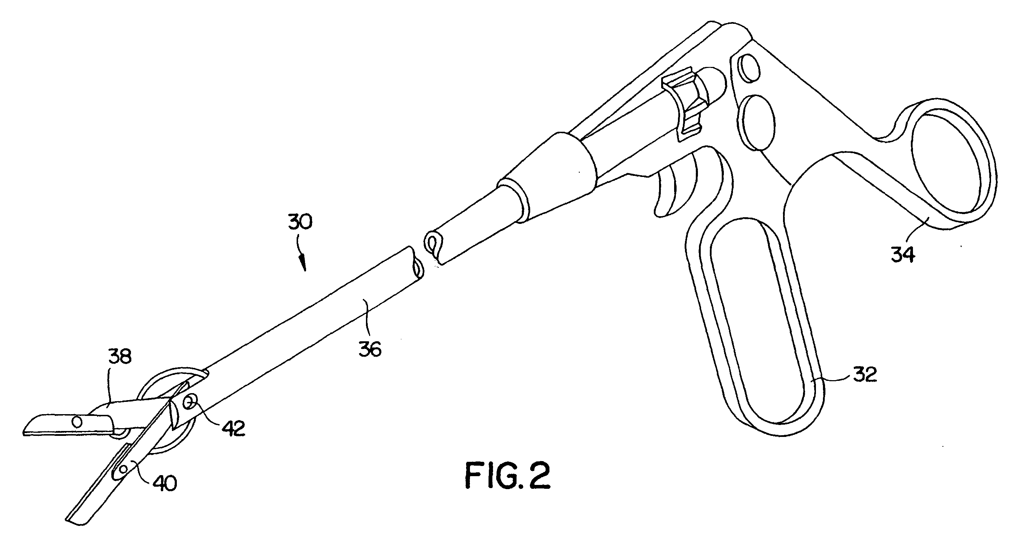

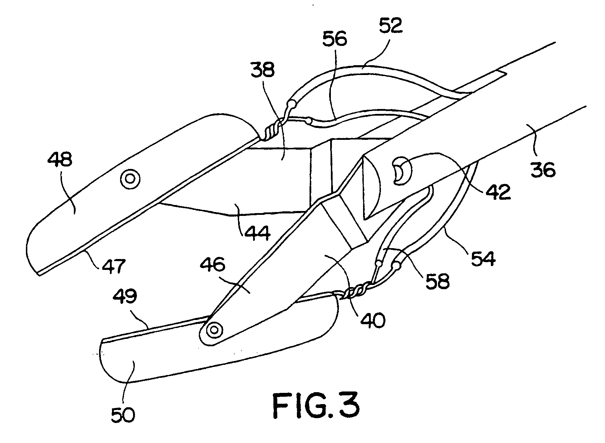

Referring to Fig. 2, a forceps 30 according to the invention is an endoscopic forceps, and includes manual handles 32, 34, an elongated shaft 36, and jaws 38, 40. The handles 32, 34 pivot together and apart and through a suitable mechanism (not shown; present in the above-mentioned prior art) control the jaws 38, 40 to also pivot together and apart about a pivot connection 42. Referring to Fig. 3, each jaw 38, 40 is formed in two parts, hinged together. The jaw 38 includes a link portion 44 connected directly to the forceps shaft 36, and the jaw 40 includes a link portion 46 also connected directly to the forceps shaft 36. A jaw portion 48 hingedly fastened to the jaw link portion 44 completes the jaw 38; a jaw portion 50 hingedly fastened to the jaw link portion 46 completes the jaw 40.

As stated in the background of the invention, a wide variety of alternatives to the structure described and shown in Fig. 2 are possible. Prominent examples from those incorporated include the structures of U.S. Patent No. 5,403,312 (Yates et al.) issued April 4, 1995; U.S. Patent No. 5,395,312 (Desai) issued March 7, 1995; and U.S. Patent No. 5,318,589 (Lichtman et al.) issued June 7, 1994

Still referring to Fig. 3, a solution supply tube 52 supplies electrolytic solution to an electrode strip 47 along the jaw portion 48, as will be described. A solution supply tube 54 supplies electrolytic solution to a similar strip 49 along the jaw portion 50. A wire 56 electrically connects to the solution supply tube 52; a wire 58 electrically connects to the solution supply tube 54. All the supplies 52, 54, 56, 58, both solution and electrical, extend from the proximal or manual handle end of the shaft 36, and connect to solution and electrical sources.

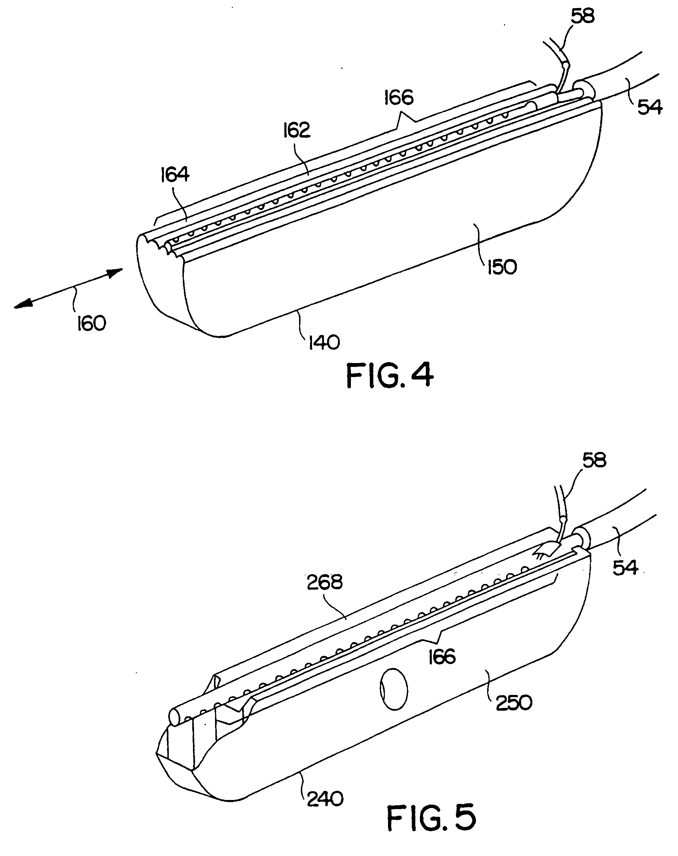

Referring to Fig. 4, and in a second form of a jaw, designated 140, a jaw portion 150 similar to jaw portion 50 in Fig. 3, includes a longitudinal dimension in the direction of arrow 160. A plurality of longitudinal grooves 162 are spaced side-by-side across the inner face 164 of the jaw portion 150. The grooves 162 extend the full longitudinal length of the jaw portion 150. The same is true of a mirror image jaw portion, not shown. Both jaw portions are incorporated in a structure as in Fig. 3, and could be placed in substitution for jaw portions 48, 50 in Fig. 3. Grooves, not shown, also preferably extend along the corresponding jaw portions 48, 50 of Figs. 2-3. Orientations of the grooves other than longitudinal are considered possible, within the limit of construction and arrangement to substantially retain solution along the operative jaw portions.

Bodily tissues to be manipulated have a natural surface roughness. This roughness significantly reduces the area of contact between the forceps jaws and manipulated tissues. Air gaps are created between conventional smooth-surfaced jaws and tissues. If the jaws were energized when dry, electrical resistance in the tissues would be increased, and the current density and tissue temperature would be extremely high. In practice, tissue surfaces are sometimes wet in spots, and yet tissue wetness is not controlled, such that electrical power is to be set on the assumption the inner jaw surfaces are dry. This assumption is necessary to minimize unwanted arcing, charring and smoke.

In contrast, in a forceps according to the invention, whether the jaw portions are grooved or smooth, whether the grooves are longitudinal or otherwise oriented, the jaw portions are uniquely formed of a material such as hollow stainless steel needle tubing such that solution infusion openings 166 may be and are formed in the jaw inner faces such as the inner face 164, as in Fig. 4. Further, the solution supplies 52, 54 shown by example in Fig. 3 may and do open into the openings 166, to supply solution to the openings 166. As most preferred, the openings 166 are laser drilled, and have a diameter in a range centered around 0.1 mm (four thousandths (0.004) of an inch), and most preferably in a range from 0.05 to 0.15 mm (two to six thousandths (0.002-0.006) inches).

The purpose of the openings 166 is to infuse solution onto and/or into the tissue adjacent to and otherwise in contact with the forceps jaw portions inner surfaces. It is understood the openings are appropriately as small in diameter as described above to assure more even flow among the openings than would otherwise occur. Further, the openings need not be so closely spaced as to mimic the surface roughness of tissues. Microporous surfaces are possibly acceptable, while they are also not necessary. Infusion of fluid through the jaws is to be maintained in a continuous flow during and throughout the application of RF energy in order for the desired tissue effect to be achieved.

With the described structure and similar structures within the scope of the invention, numerous advantages are obtained. Deeper and quicker coagulation is possible. The conductive solution infused onto and into the tissues maintains relatively consistent maximal electrical contact areas, substantially preventing hot spots and allowing higher power than soft coagulation. Little to no arcing, cutting smoke or char is formed. . Jaw and tissue surface temperatures are lower than otherwise, resulting in significantly less adhesion of tissue to jaw surfaces, and substantially no desiccation. One mode of coagulation may be used in the place of the three modes soft, forced, and spray. Coagulation is possible of even the most challenging oozing tissues such as lung, liver and spleen tissues. Coagulation is more precise, where other coagulation modes sometimes spark to the sides and produce coagulation where not desired.

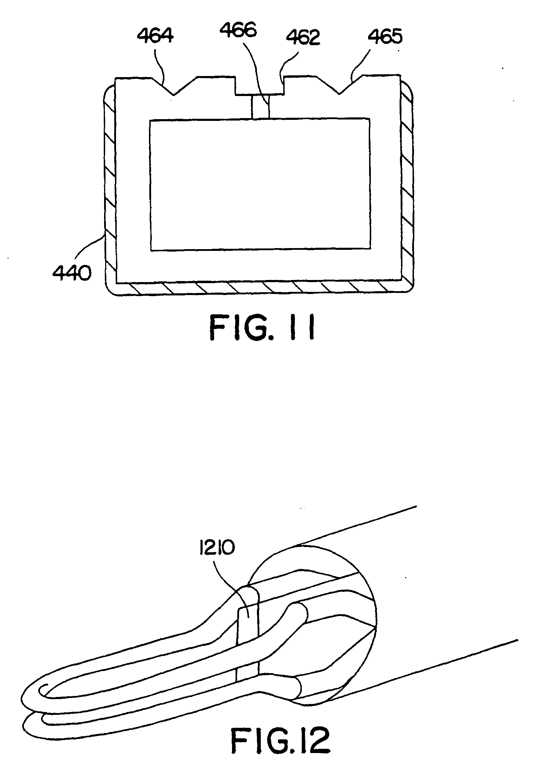

Also, and importantly, electrosurgical cutting by desiccation may be avoided, and tissue sealing achieved. As desired, tissue sealing may occur alone, or be accompanied with mechanical cutting, as by a retractable and advancable blade as in U.S. Patent No. 5,458,598, and as with blade 1210 in Fig. 12, or otherwise. The tissue sealing itself is understood to occur by flow of electrolytic solution to the manipulating portions of the forceps in combination with energization of the solution with electrical energy, and when included, in combination with pressure on, or compression of, the tissue. Compression of tissue is understood to deform tissues into conditions of sealing of tissues and especially vascalature. Compression of tissue followed by application of solution and energy is understood to permanently maintain compressed deformation of tissue, when present, and to shrink tissue and cause proteins to fix in place. Additional understanding of others is provided in the Yates et al. patent referenced above.

The body of the forceps itself may or not be energized. As most preferred, the solution primarily provides the beneficial functions and effects of the instrument. The effectiveness and extent of the tissue sealing is a function primarily of the type of tissue being manipulated, the quantity of electrolytic solution supplied to the tissue, and the power of the electrical energy supplied to the solution. Tissues not previously considered to be suitable for manipulation, as by cutting, are rendered suitable for manipulation by being sealed against flow of fluids, including bodily fluids and air. With the invention, for example, lung tissue may be cut after sealing, with the tissue adjacent the sealed tissue retaining blood and air. Examples of the principal parameters of specific uses of the invention are provided in the following table. It is understood that the combined consequences of the parameters are that energy density in the tissue to be treated is in a range to effect sealing of the tissue. However, in general, a power output of 7 to 150 watts is preferred.

| Fluid Quantity | Power | Tissue | Effect |
|---|---|---|---|
| 2 cc=s per minute per electrode | 20 watts for 30 seconds | 1 cm diameter vessel | hemostasis through the vessel |
| | | | |
| 2 cc=s per minute per electrode | 30 watts for 45 seconds | lung tissue | hemostasis and aerostasis |
| | | | |
| 4 cc=s per minute per electrode | 40 watts for 90 seconds | 2 cm thickness liver tissue | hemostasis |

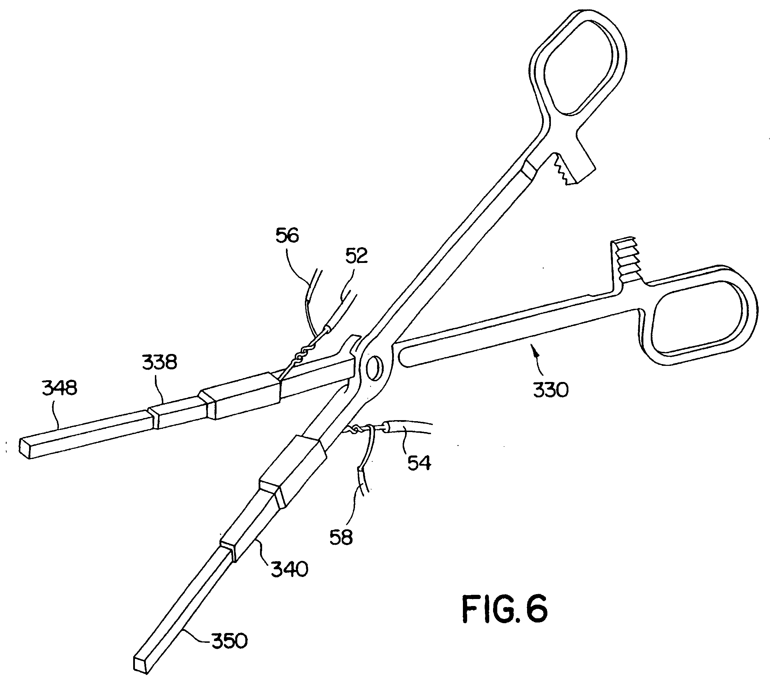

In the examples for which the table is provided, the electrolytic solution is saline. In the first example, the device in use was a device as in Fig. 2, with electrodes of 16 gauge tubing, 1 cm long. The tool in use in the second and third examples was a forceps as in Fig. 6, with jaw portions 348, 350, to be described, 4 mm wide and 2.8 cm long. No desiccation was observed at the tissue/electrode interface. The device of Fig. 2 is preferred for vessel closure.

A wide variety of the currently installed electrosurgical generators could and will provide proper waveforms and power levels for driving the described forceps. The waveforms need only be sine waves at about 500 kHz, and the power need only be about 30 or more watts. As example of available generators, Valleylab generators are acceptable and widely available.

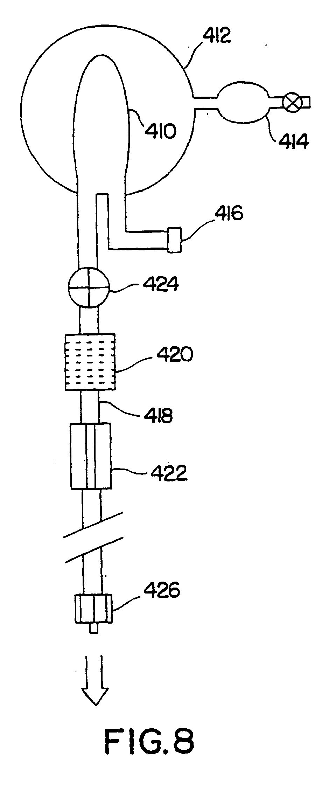

The electrolytic solution supplied to the forceps need only be saline, although a variety of non-toxic and toxic electrolytic solutions are possible. Toxic fluids may be desirable when excising undesired tissues, to prevent seeding during excision. Use of a pressure bulb is possible, as shown in Fig. 8. A flexible reservoir such as an intravenous (IV) bag 410 is surrounded with a more rigid rubber bulb 412 that is pressurized with air through an attached squeeze bulb 414. The reservoir is filled with solution through an injection port 416. An outflow line 418 has a filter 420 and a capillary tube flow restrictor 422 to meter flow. A clamp or valve 424 and connector 426 are also provided. A typical flow rate is one to two (1-2) cc/min at a maximum pressure of approximately sixteen pounds per square inch (16 psi) (52 mmHg). An example of opening diameters, numbers, and flow rate is as follows: opening diameter, 0.16 mm; number of openings, 13 per cm; and flow rate, 2 cc's per minute. A long slit has also been used and found acceptable. In this embodiment, flow rates of 0.01 to 50 cc/min are preferred.

It is understood that highly significant to the invention is the spacing of a plurality of solution openings along the jaw inner surfaces. Single openings as in Ohta et al., that effectively pour fluid adjacent one portion of forceps, are generally not considered suitable or effective. Openings along outer surfaces of the jaws, opposite inner surfaces, are also generally not considered suitable or effective.

Referring to Figs. 4 and 5, the configurations of the most preferred solution openings are disclosed. Referring to Fig. 5, in a jaw 240, longitudinally spaced openings 166 are rotated from those shown in Fig. 4, in a jaw portion 250, to turn the openings away from most direct contact with tissues, and more carefully eliminate any unintended plugging of the openings. Electrical insulators 268 in the form of elongated strips extend alongside the tubes which include the openings 166.

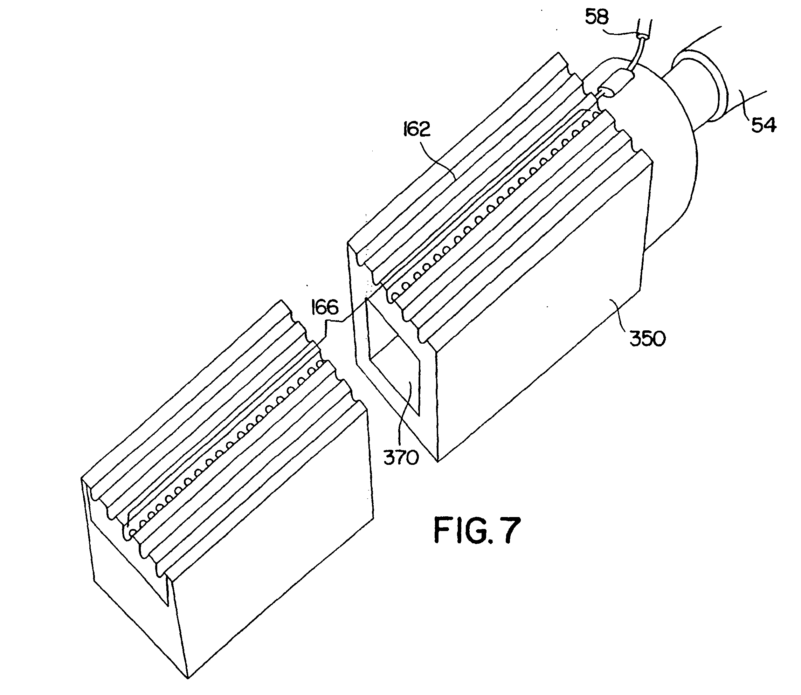

Referring to Fig. 6, open surgical forceps 330 include jaws 338, 340 with jaw portions 348, 350. As with jaw portion 350 in Fig. 7, the jaw portions 348, 350 include spaced solution infusion openings 166 in the central longitudinal groove of a plurality of grooves 162. A central channel 370 of both jaw portions 348, 350, as shown relative to jaw portion 350 in Fig. 7, supplies solution to the openings 166 from solution supplies 52, 54. As with the endoscopic forceps of Figs. 2-5, the open surgical forceps 330 benefits from the unique enhancement of electrosurgical functions through the infusion of electrolytic solutions onto and into tissues through the spaced, laser drilled, solution infusion openings in the grooves 162.

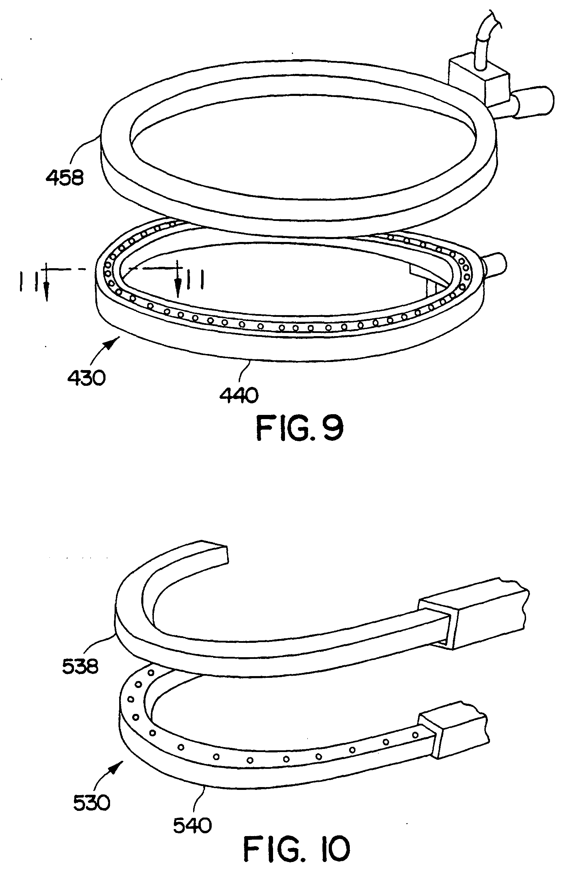

Referring to Figs. 9 and 10, open surgical devices 430 and 530 also include jaws 438, 440 and 538, 540, respectively. The jaw portions of these devices are curved, and in the case of device 430, circular, to adapt the invention to specialized surgical situations of tissue manipulation, such as those in which fluid flow is to be terminated all around a tissue to be isolated and resected or excised. An example of such a tissue is a lesion or tumor of lung tissue. In endoscopic or open surgery, such lesions or tumors may be encircled and/or isolated, surrounding tissue sealed, and the lesions or tumors thereafter resected. Preferably, a one centimeter margin is resected about any lesion or tumor, with the lesion or tumor. As shown, the devices 430, 530 are formed of substantially square cross-section tubing, best shown in the cross-sectional drawing of Fig. 11. As most preferred, the tubing incorporates a central, depressed, cross-sectionally rectangular, and elongated groove 462 and equilaterally spaced, cross-sectionally triangular, parallel, and elongated outer grooves 464, 465. Laser drilled openings 466, similar to openings 166 described above, are located in and spaced along the central groove 462.

Alternative cross-sectional shapes of tubing may be employed, as exemplified in Fig. 12. Flatter operative, e.g., inner faces of tubing are preferred within limits of constructing and arranging the operative faces to facilitate firm grasping and holding of tissue. Non-operative surfaces, being less of concern, may adapt to a variety of contours for a variety of alternative reasons. Further, malleable tubing may be employed, to permit the surgeon to shape the operative portions of the invented devices to specific physiological situations.

The infusion of conductive solutions, referred to here also as electrolytic solutions, simultaneously with the application of RF energy to tissues is discussed in further detail in U.S. Patent No. 5,431,649 entitled "Method and Apparatus for R-F Ablation" in the name of Peter M.J. Mulier and Michael F. Hoey; in U.S. Patent No. 5,609,151, entitled "Method and Apparatus for R-F Ablation" in the name of Peter M.J. Mulier.

The preferred embodiments, and the processes of making and using them, are now considered to be described in such full, clear, concise and exact terms as to enable a person of skill in the art to make and use the same. The above description is given by way of example only. For example, if the invented device is incorporated in forceps, the forceps may be varied in a range from excision and cutting biopsy forceps, to endoscopic forceps, dissecting forceps, and traumatic, atraumatic and flexible endoscopic grasping forceps. The jaws may close into full and tight contact with each other, or close into spaced relationship to each other, to accommodate tissue for purposes other than cutting. As expressed above, parallel spaced relationship is considered most preferable for uniformity of application of pressure across tissue to be affected.

A variety of features such as jaw serrations, single acting and double acting jaws, closing springs, ratchet locks, fingertip rotation rings, color coding and smoke aspiration may or may not be included with the features described in detail. Devices according to the invention may be constructed and arranged to grasp, hold, fix, cut, dissect, expose, remove, extract, retrieve, and otherwise manipulate and treat organs, tissues, tissue masses, and objects. Endoscopic forceps according to the invention may be designed to be used through a trocar. Bipolar and monopolar currents may both be used. With monopolar current, grounding pads may be placed under patients. The described grooves may be eliminated in favor of alternative grooves.

For purposes of the appended claims, the term "manipulate" includes the described functions of grasping, holding, fixing, cutting, dissecting, exposing, removing, extracting, retrieving, coagulating, ablating and otherwise manipulating or similarly treating organs, tissues, tissue masses, and objects. Also for purposes of the appended claims, the term "tissue" includes organs, tissues, tissue masses, and objects. Further for purposes of the appended claims, the term "electrical energy sufficient to affect tissue" includes electrical energy sufficient to raise tissue temperature to cause non-reversible effect on tissue as described above.

## Claims

1. An electrosurgical medical device, comprising in combination:
co-operating jaws (38, 40) each including a jaw portion (48, 50) for manipulating tissue;
solution supply means (52, 54) for supplying electrolytic solution to the jaw portions (48, 50);
solution infusion openings (166) defined along the jaw portions of the jaws (38, 40), the solution infusion openings being constructed and arranged for receiving an electrically conductive solution from the solution supply means and infusing the solution through the openings and along the jaw portions into tissue manipulated by the jaw portions; and
an electrical conductor (56, 58) electrically connected to the solution supply means for conducting electrical energy to the electrically conductive solution in contact with the manipulated tissue.

2. An electrosurgical medical device as in claim 1, the jaw portions (48, 50) including opposed inner faces (164), the solution infusion openings (166) being a plurality of openings defined and spaced along the inner faces.

3. An electrosurgical medical device as in claim 1 or 2, further comprising:
at least one groove (162) along at least one of said jaw portions, the solution infusion openings in the at least one jaw portion having at least one opening located adjacent the at least one groove.

4. An electrosurgical medical device as in claim 1 or 2, further comprising:
at least one groove along at least one of the jaw portions, the solution infusion openings in the at least one jaw portion having at least one opening located in the at least one groove.

5. An electrosurgical medical device as in claim 1 or 2, further comprising a plurality of grooves (162) along the jaw portions of the co-operating jaws, the solution infusion openings located in the plurality of grooves.

6. An electrosurgical medical device as in any preceding claim further comprising:
serrations on at least one of the jaw portions.

7. An electrosurgical medical device as in any preceding claim further comprising:
a proximal manual handle (32, 34), a shaft (36) extending from the handle to the jaws (38, 40), whereby the jaws are distally located, and a mechanism connected to the handle and jaws whereby the proximal handle manipulates the distal jaws, for endoscopic use of the device.

8. An electrosurgical medical device as in any preceding claim further comprising:
a pivot (42) for pivoting movement of the jaws (38, 40) into contact with each other and away from contact with each other.

9. An electrosurgical medical device as in claim 8 further comprising:
a blade (1210) along at least one of said jaw portions, for mechanically cutting tissue as the jaw portions are pivoted toward contact with each other.

10. An electrosurgical medical device as in any preceding claim, further comprising:
said jaws including means for substantially uniformly compressing tissue being manipulated by the jaw portions.

11. An electrosurgical medical device as in any preceding claim, further comprising
an electrical supply for supplying said electric energy to the solution.

12. An electrosurgical medical device as claimed in claim 1, further comprising
a plurality of longitudinal grooves (162) along said jaw portions;
a pivot (42) for pivoting movement of said jaws into contact with each other and away from contact with each other;
said solution supply means (52, 54) being capable of supplying said solution at a rate in a range of .01 to 100 cc/min; and
an extended shaft (36) connected to said jaws for endoscopic use of said device; and wherein said plurality of solution infusion openings (166) being spaced along each of the jaw portions and located in said plurality of longitudinal grooves.

## Patentansprüche

1. Elektrochirurgische medizinische Vorrichtung, folgendes in Kombination umfassend:
Zusammenwirkende Klemmbacken (38, 40), wobei jede einen Backenabschnitt (48, 50) für das Manipulieren von Gewebe einschließt;
Lösungsversorgungsmittel (52, 54) für das Liefern einer elektrolytischen Lösung an die Backenabschnitte (48, 50);
Lösungsinfusionsöffnungen (166), definiert entlang der Backenabschnitte der Klemmbacken (38, 40), wobei die Lösungsinfusionsöffnungen derart konstruiert und angeordnet sind, dass sie eine elektrisch leitende Lösung von den Lösungsversorgungsmitteln empfangen und die Lösung durch die Öffnungen und entlang der Backenabschnitte in das Gewebe einflößen, das von den Backenabschnitten manipuliert wird; und
einen elektrischen Leiter (56, 58) elektrisch mit den Lösungsversorgungsmitteln verbunden, um elektrische Energie zu der elektrisch leitenden Lösung zu leiten, die sich in Kontakt mit dem manipulierten Gewebe befindet.

2. Elektrochirurgische medizinische Vorrichtung nach Anspruch 1, bei welcher die Backenabschnitte (48, 50) gegenüberliegende innere Flächen (164) einschließen, wobei die Lösungsinfusionsöffnungen (166) eine Vielzahl von Öffnungen sind, die entlang der inneren Flächen definiert und verteilt sind.

3. Elektrochirurgische medizinische Vorrichtung nach Anspruch 1 oder 2, ferner umfassend:
zumindest eine Nut (162) entlang zumindest eines der Backenabschnitte, wobei die Lösungsinfusionsöffnungen in dem zumindest einen Backenabschnitt zumindest eine Öffnung aufweisen, die benachbart zu der zumindest einen Nut angeordnet ist.

4. Elektrochirurgische medizinische Vorrichtung nach Anspruch 1 oder 2, ferner umfassend:
zumindest eine Nut entlang zumindest eines der Backenabschnitte, wobei die Lösungsinfusionsöffnungen in dem zumindest einen Backenabschnitt zumindest eine Öffnung aufweisen, die in der zumindest einen Nut angeordnet ist.

5. Elektrochirurgische medizinische Vorrichtung nach Anspruch 1 oder 2, ferner umfassend eine Vielzahl von Nuten (162) entlang der Backenabschnitte der zusammenwirkenden Klemmbacken, wobei die Lösungsinfusionsöffnungen in der Vielzahl der Nuten angeordnet sind.

6. Elektrochirurgische medizinische Vorrichtung nach einem der vorherigen Ansprüche, ferner umfassend:
Kerbverzahnungen an zumindest einem der Backenabschnitte.

7. Elektrochirurgische medizinische Vorrichtung nach einem der vorherigen Ansprüche, ferner umfassend:
einen proximalen Handgriff (32, 34), einen Stiel (36), der sich vom Griff zu den Klemmbacken (38, 40) erstreckt, wobei die Klemmbacken distal angeordnet sind, sowie einen Mechanismus, verbunden mit dem Griff und den Klemmbacken, wobei der proximale Griff die distalen Klemmbacken manipuliert, für eine endoskopische Verwendung der Vorrichtung.

8. Elektrochirurgische medizinische Vorrichtung nach einem der vorherigen Ansprüche, ferner umfassend:
eine Drehachse (42) für eine Schwenkbewegung der Klemmbacken (38, 40) in gegenseitigen Kontakt und aus gegenseitigem Kontakt heraus.

9. Elektrochirurgische medizinische Vorrichtung nach Anspruch 8, ferner umfassend:
eine Klinge (1210) entlang zumindest eines der Backenabschnitte für das mechanische Schneiden von Gewebe, wenn die Backenabschnitte in Richtung des gegenseitigen Kontakts geschwenkt werden.

10. Elektrochirurgische medizinische Vorrichtung nach einem der vorherigen Ansprüche, ferner umfassend:
wobei die Klemmbacken Mittel für das im Wesentlichen gleichförmige Zusammendrücken von Gewebe, das von den Backenabschnitten manipuliert wird, einschließen.

11. Elektrochirurgische medizinische Vorrichtung nach einem der vorherigen Ansprüche, ferner umfassend:
eine elektrische Versorgung für die Lieferung der elektrischen Energie an die Lösung.

12. Elektrochirurgische medizinische Vorrichtung nach Anspruch 1, ferner umfassend:
eine Vielzahl von Längsnuten (162) entlang der Backenabschnitte;
eine Drehachse (42) für eine Schwenkbewegung der Klemmbacken in gegenseitigen Kontakt und aus gegenseitigem Kontakt heraus;
wobei die Lösungsversorgungsmittel (52, 54) geeignet sind, die Lösung in einer Rate zu liefern, die im Bereich von 0,01 bis 100 cm³ / Minute liegt; und
einen verlängerten Stiel (36), verbunden mit den Klemmbacken, für die endoskopische Verwendung der Vorrichtung; und wobei
eine Vielzahl von Lösungsinfusionsöffnungen (166) entlang jedes der Backenabschnitte verteilt ist und in der Vielzahl an Längsnuten angeordnet ist.

## Revendications

1. Dispositif médical électrochirurgical comprenant en combinaison:
- des mâchoires coopérantes (38, 40), chacune comprenant une portion mâchoire (48, 50) pour manipuler le tissu ;
- moyen d'alimentation en solution (52, 54) pour acheminer une solution électrolytique aux portions mâchoire (48, 50) ;
- des orifices pour infusion de solution (166) définies au long des portions mâchoire des mâchoires (38, 40), les orifices pour infusion de solution étant construites et arrangées de façon à recevoir une solution électriquement conductrice en provenance du moyen d'alimentation en solution et infuser ladite solution par les orifices et au long des portions mâchoire dans le tissu manipulé par les portions mâchoire ; et
- un conducteur électrique (56, 58) électriquement connecté au moyen d'alimentation en solution pour conduite l'énergie électrique à la solution électriquement conductrice en contact avec le tissu manipulé.

2. Dispositif médical électrochirurgical selon la revendication 1, les portions mâchoire (48, 50) comprenant des faces intérieures (164) opposées l'une à l'autre, les orifices pour infusion de solution (166) étant une pluralité d'orifices définies et espacées au long des faces intérieures.

3. Dispositif médical électrochirurgical selon la revendication 1 ou 2, comprenant en outre :
au moins une rainure (162) au long au moins une desdites portions mâchoire, les orifices pour infusion de solution dans au moins une portion mâchoire ayant au moins un orifice se trouvant juxtaposé à au moins une rainure.

4. Dispositif médical électrochirurgical selon la revendication 1 ou 2, comprenant en outre :
au moins une rainure au long au moins une desdites portions mâchoire, les orifices pour infusion de solution dans au moins une portion mâchoire ayant au moins un orifice se trouvant dans au moins une rainure.

5. Dispositif médical électrochirurgical selon la revendication 1 ou 2, comprenant en outre une pluralité de rainures (162) au long des portions mâchoire des mâchoires coopérantes, les orifices pour infusion de solution se trouvant dans la pluralité de rainures.

6. Dispositif médical électrochirurgical selon une quelconque des revendications précédentes, comprenant en outre :
des dentelures sur au moins une des portions mâchoire.

7. Dispositif médical électrochirurgical selon une quelconque des revendications précédentes, comprenant en outre :
une manche manuelle proximale (32, 34) , une tige (36) s'étendant de la manche aux mâchoires (38, 40), **caractérisée en ce que** les mâchoires se trouvent en position distale, et un mécanisme connecté à la manche et aux mâchoires, **caractérisé en ce que** la manche proximale manipule les mâchoires distales, pour l'utilisation endoscopique du dispositif.

8. Dispositif médical électrochirurgical selon une quelconque des revendications précédentes, comprenant en outre :
un pivot (42) pour un mouvement pivotant des mâchoires (38, 40) destiné à les faire entrer en contact l'une avec l'autre, et un mouvement, pour les éloigner l'une de l'autre.

9. Dispositif médical électrochirurgical selon la revendication 8, comprenant en outre :
une lame (1210) au long au moins une desdites portions mâchoire, pour couper mécaniquement le tissu lorsque les portions mâchoire sont pivotées pour les faire entrer en contact l'une avec l'autre.

10. Dispositif médical électrochirurgical selon une quelconque des revendications précédentes, comprenant en outre :
lesdites mâchoires comprenant des moyens pour comprimer, de façon essentiellement uniforme, le tissu manipulé par les portions mâchoire.

11. Dispositif médical électrochirurgical selon une quelconque des revendications précédentes, comprenant en outre :
une alimentation électrique pour alimenter la solution de ladite énergie électrique

12. Dispositif médical électrochirurgical selon la revendication 1, comprenant en outre :
une pluralité de rainures longitudinales (162) au long desdites portions mâchoires ;
un pivot (42) pour un mouvement pivotant desdites mâchoires destiné à les faire entrer en contact l'une avec l'autre et à les éloigner l'une de l'autre ;
ledit moyen d'alimentation en solution (52, 54), capable de fournir ladite solution à un taux allant de 0,01 à 100 cc/min ;
une tige prolongée (36) connectée aux dites mâchoires pour l'usage endoscopique dudit dispositif ; et dans lequel
ladite pluralité d'orifices pour infusion de solution (166) sont espacés au long de chacune des portions mâchoire se trouvant dans ladite pluralité de rainures longitudinales.
